# EUROPEAN PATENT APPLICATION

(11) **EP 2 529 717 A1**
(43) Date of publication of application: **05.12.2012**
(21) Application number: 12730355.0
(22) Date of filing: 04.04.2012
(51) Int. Cl.: A61F 13/49, A61F 13/42, A61F 13/514, A61F 13/53

(54) **DISPOSABLE ABSORBENT ARTICLE**

(30) Priority: 04.04.2011 JP 2011083201
(71) Applicant: Unicharm Corporation, Ehime 799-0111 (JP)
(72) Inventor: ICHIKAWA, Makoto, Kanonji-shi, Kagawa 769-1602 (JP); SASAYAMA, Kenichi, Kanonji-shi, Kagawa 769-1602 (JP); KATSURAGAWA, Kunihiko, Kanonji-shi, Kagawa 769-1602 (JP)
(74) Representative: Peter, Julian
(86) International application number: PCT/JP2012/059170
(87) International publication number: WO 2012/137809

(57) **Abstract**

A disposable absorbent article improved so that occurrence of defecation may be easily perceived from the outside. A diaper 1 as one example of a disposable absorbent article includes a crotch inner sheet 28 lying on a skin-facing surface, a crotch outer sheet 29 lying on a non-skin-facing surface and a liquid-absorbent structure 80 interposed between these inner and outer sheets 28, 29. In this diaper 1, a total luminous transmittance from the outer sheet 29 through the liquid-absorbent structure 80 to the inner sheet 28 is in a range of 15% to 65%. The liquid-absorbent structure 80 has a longitudinal direction Y, a transverse direction X and a thickness direction W and includes a first sheet 81 and a second sheet 82 overlapping each other and absorbent polymer particles 84 interposed between these first and second sheets 81, 82 wherein the liquid-absorbent structure 80 is divided into absorbing regions 85 in which the absorbent polymer particles 84 are present and sealing regions 86 in which none of the absorbent polymer particles 84 is present arranged alternately in the in the longitudinal direction Y. In the sealing regions 86, the first sheet 81 and the second sheet 82 are bonded to each other.

## Description

### {Technical Field}

This invention relates to disposable absorbent articles and more particularly to disposable absorbent articles such as diapers, toilet-training pants and incontinent briefs.

Conventionally, disposable absorbent articles including a liquid-permeable inner sheet lying on the side facing the wearer's skin, a liquid-impermeable outer sheet lying on the side facing away from the wearer's skin and an liquid-absorbent core interposed between these inner sheet and outer sheet are well known.

Forexample, JP2005-261466A (PTL1) discloses an absorbent article having a moisture indicator disposed between the outer sheet and the absorbent core. When the wearer of this absorbent article urinates, the moisture indicator changes its initial color and thereupon a third person, for example, a care personnel may visually recognize occurrence of urination on this absorbent article from the outside of this article.

### {Citation List}

### {Patent Literature}

{PTL 1} JP 2005-261466 A

### {Summary}

### {Technical Problem}

In the absorbent article disclosed in JP 2005-261466 A, it is certainly possible for the third person to perceive occurrence of urination on the basis of color change appearing on the moisture indicator. However, if no color change appears on the moisture indicator in spite of occurrence of defecation, the third person may not perceive it. To make sure whether defecation has occurred or not on the absorbent article, the third person must move his or her nose closer to the absorbent article to sniff out feces or broaden the waist-opening against contractile force of the elastic members attached to the periphery of the waist-opening.
If feces are left within the absorbent article for a long time period, the wearer will experience an uncomfortable feeling and, in addition, the wearer's skin might be damaged in the form of eruption or the like.

An object of this invention is to provide a disposable absorbent article improved so that occurrence of defecation may be easily perceived from the outside.

### {Solution to Problem}

This invention provides a disposable absorbent article including an inner sheet lying on a skin-facing surface, an outer sheet lying on a non-skin-facing surface and a liquid-absorbent structure interposed between these inner and outer sheets.

In this absorbent article, a total luminous transmittance from the outer sheet through the liquid-absorbent structure to the inner sheet is in a range of 15% to 65%.

According to one embodiment of this invention, the liquid-absorbent structure has a longitudinal direction, a transverse direction and a thickness direction and includes:
absorbing regions containing absorbent polymer particles between a first sheet and a second sheet and being arranged alternately in the longitudinal direction; and
sealing regions surrounding each of the absorbing regions and being defined by sealing the first and second sheet.

According to another embodiment of this invention, the liquid-absorbent structure has a dimension in the thickness direction in a range of 0.5mm to 9.0mm and has a bending resistance in the longitudinal direction measured by a cantilever method is in a range of 15 to 140mm. Measurement of the bending resistance is conducted on the skin-facing surface and the non-skin-facing surface of respective samples (50mm in the transverse direction X, 150mm in the longitudinal direction Y) cut out from a crotch member 30 of a diaper 1 in the vicinity of transverse imaginary center line Q so as to include no fold line, in accordance with Bending Resistance Test Method A (45 Degree Cantilever Method) of JIS L1096 Section 8.19.1.

According to still another embodiment of this invention, the absorbent article is provided in the form of pants-type article including an annular waist member, the inner sheet, the outer sheet, the liquid-absorbent structure and a crotch member bonded to the non-skin-facing surface of the waist member.

According to yet another embodiment of this invention, the crotch member includes a pair of side flaps and a pair of front and rear end flaps respectively extending along opposite side edges and opposite ends of at least one of the inner sheet and the outer sheet and the side flaps are folded onto the skin-facing surface; and
the side flaps are continuously coated in the longitudinal direction with adhesives with which the crotch member is bonded to the waist member and thereupon a rear pocket may be formed between the waist member and the crotch member.

According to further another embodiment of this invention, unpatterned white fibrous nonwoven fabric is used as material for the inner sheet and the outer sheet and unpatterned white fibrous nonwoven fabric is used also as material for a waist outer sheet lying on the non-skin-facing surface.

### {Advantageous Effects of Invention}

The disposable absorbent article according to this invention has a total luminous transmittance from the outer sheet through the liquid-absorbent structure to the inner sheet is in a range of 15% to 65% and therefore feces having been discharged onto the skin-facing surface of the inner sheet may be visually perceived through the outer sheet. In this way, occurrence of detectionmaybe easily perceived from the outside of the absorbent article.

### {Brief Description of Drawings}

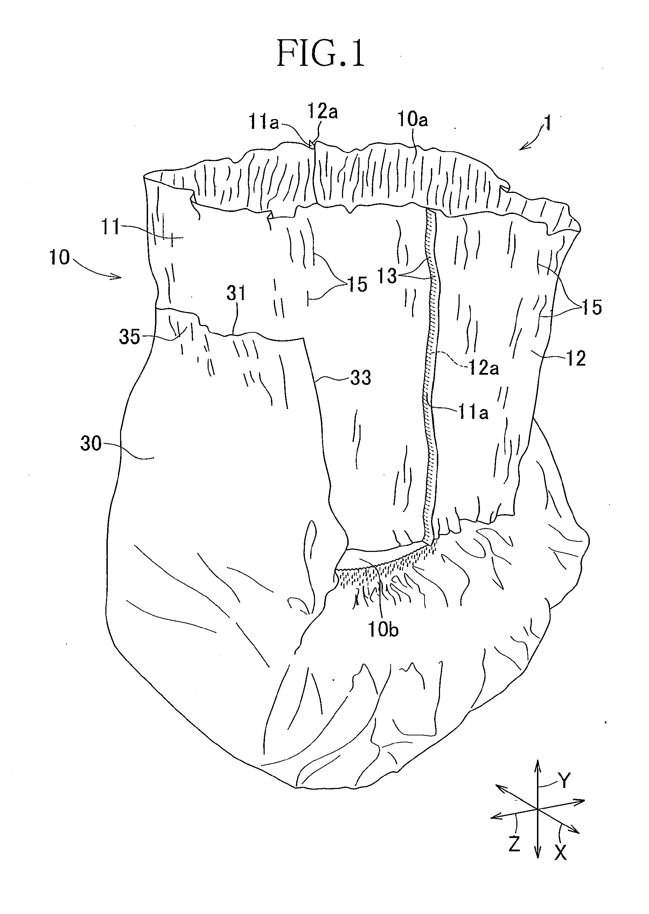

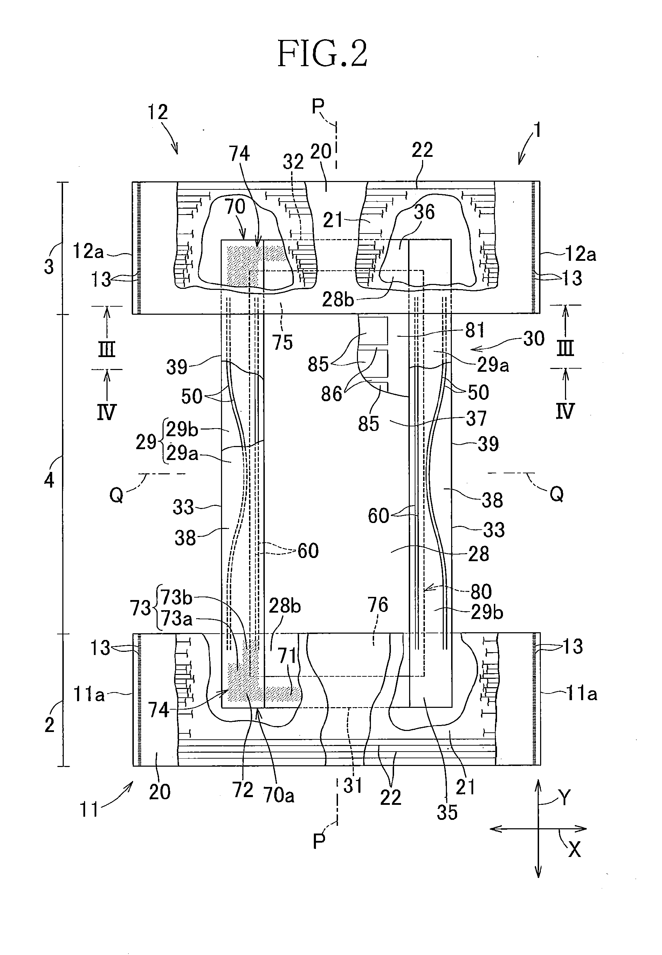

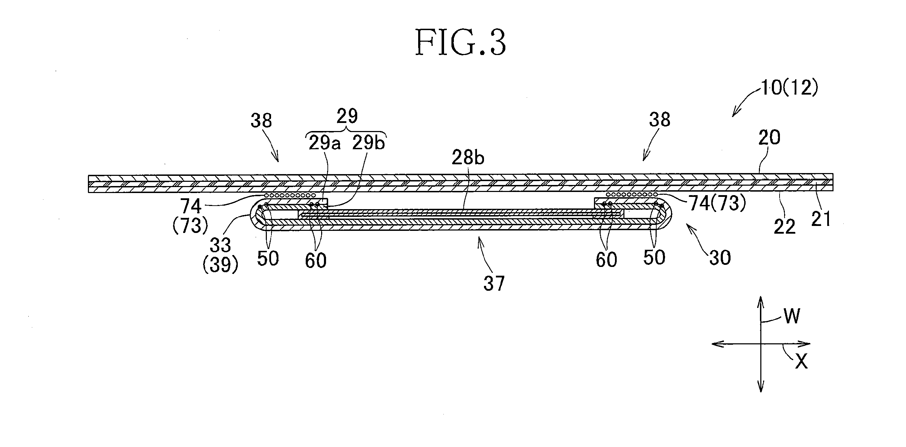

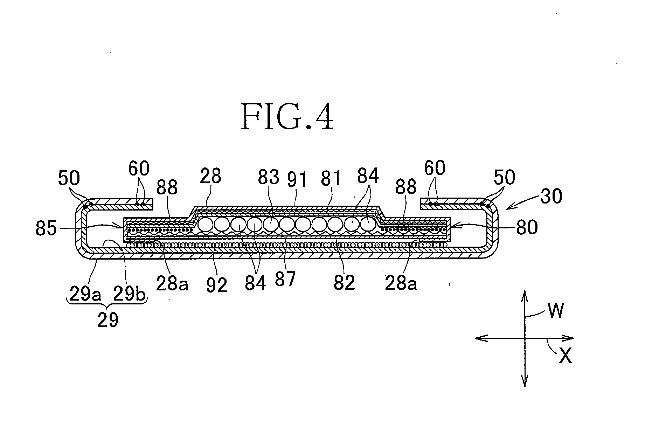

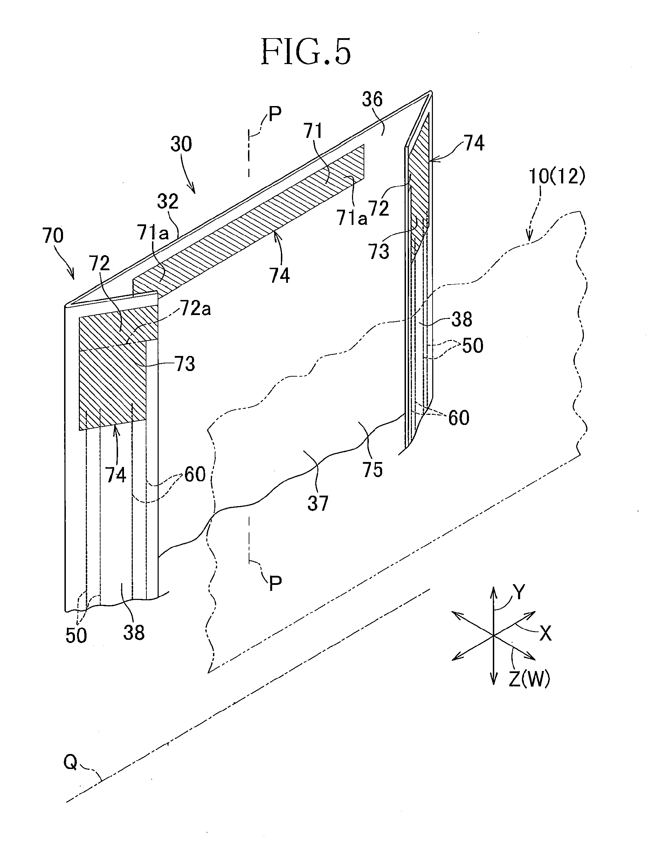

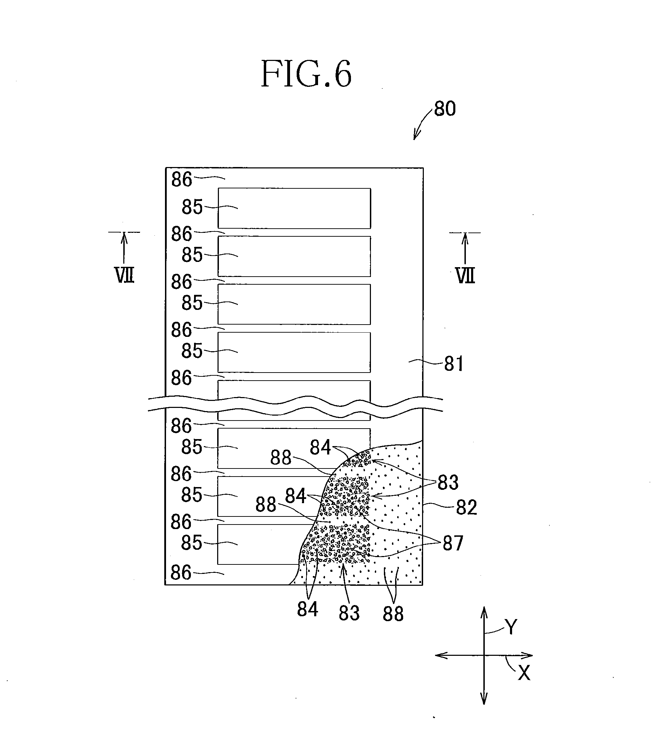

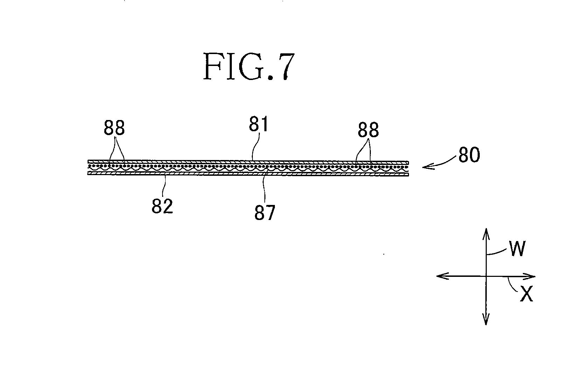

{Fig. 1} Fig. 1 is a perspective view of a disposable diaper as an example of disposable absorbent articles according to this invention.
{Fig. 2} Fig. 2 is a partially cutaway developed plan view of the diaper as viewed from its side facing the wearer's skin.
{Fig. 3} Fig. 3 is a sectional view taken along line III-III in Fig. 2.
{Fig. 4} Fig. 4 is a sectional view taken along-line IV-IV in Fig. 2.
{Fig. 5} Fig. 5 is a perspective view illustrating a manner in which a waist member and a crotch member are joined to each other.
{Fig. 6} Fig. 6 is a plan view of a liquid-absorbent structure.
{Fig. 7} Fig. 7 is a sectional view taken along line VII-VII in Fig. 6.

### {Description of Embodiments}

An embodiment of a disposable diaper as an example of disposable absorbent articles according to this invention will be described hereunder with reference to the accompanying drawings.

Fig. 1 is a partially cutaway perspective view illustrating a pants-type diaper 1 having a waist-opening 10a and a pair of leg-openings 10b as viewed from the front thereof.
In Fig. 1, X indicates a transverse direction, Y indicates a longitudinal direction which is orthogonal to the transverse direction X and Z indicates a front-back direction which is orthogonal to the transverse direction X and to the longitudinal direction Y.
The diaper 1 includes an annular waist member 10 extending in a waist-circumferential direction and a crotch member 30 extending in a direction intersecting with the waist-circumferential direction. The waist member 10 has a front panel 11 and a rear panel 12 respectively lying at front and rear portions in the front-back direction Z and respective opposite side edges 11a, 12a in the transverse direction X of these front and rear panels 11, 12 are put flat and joined together at seams 13 arranged intermittently in the longitudinal direction Y.

Fig. 2 is a partially cutaway plan view of the diaper 1 developed in the transverse direction X and the front-back direction Z after the front and rear panels are peeled off from each other along arrays of the seams 13 and illustrates the surface of the diaper 1 opposed to the wearer's skin (referred to hereinafter simply as a skin-facing surface and the surface opposite to this skin-facing surface will be referred to hereinafter simply as a non-skin-facing surface). In Fig. 2, P indicates a longitudinal imaginary center line extending in the longitudinal direction Y to bisect the diaper 1 in the transverse direction X and Q indicates a transverse imaginary center line extending in the transverse direction X to bisect the crotch member 30 in the longitudinal direction Y. Fig. 3 is a sectional view taken along line III-III in Fig. 2. Fig. 4 is a sectional view taken along line IV-IV in Fig. 2. A thickness direction W in Figs. 3 and 4 corresponds to the front-back direction Z in Fig. 1.

The diaper 1 has a front waist region 2, a rear waist region 3 and a crotch region 4 extending between the front and rear waist regions 2, 3 wherein these regions are continuous in the longitudinal direction Y. The front waist region 2 is adapted to cover the front waist of the wearer, the rear waist region 3 is adapted to cover the wearer's rear waist and the crotch region 4 is adapted to cover the wearer's crotch region.

The front and rear waist regions 2, 3 are defined by the front and rear waist panels 11, 12 and the crotch region 4 is defined by the crotch member 30. As illustrated, the front panel 11, the rear panel 12 and the crotch member 30 are symmetric about the longitudinal imaginary center line P, respectively.

The front and rear waist panels 11, 12 respectively include waist inner sheets 20 lying on the skin-facing surface, waist outer sheets 21 lying on the non-skin-facing surface and waist elastic members 22 interposed between the waist inner and outer sheets 20, 21. As material for the waist inner and outer sheets 20, 21, air-permeable, unpatterned white fibrous nonwoven fabric is used. The waist inner and outer sheets 20, 21 are bonded to each other with hot melt adhesives (not shown).

The waist elastic members 22 are secured under tension to at least one of the waist inner and outer sheets 20, 21 with hot melt adhesives (not shown). In Fig. 1, the waist elastic members 22 are left contract in the transverse direction X and under contractile force of the waist elastic members 22, the waist inner and outer sheets 20, 21 are formed with the gathers 15. In this regard, to fix the elastic members 22 to the waist inner and outer sheets 20, 22, instead of coating the elastic members 22 with adhesive, at least one of the waist inner and outer sheets 20, 21 may be coated with adhesives or instead of coating the waist inner and outer sheets 20, 21, the elastic members 22 may be coated with adhesive or both the waist inner and outer sheets and the elastic members 22 may be coated with adhesives.

As illustrated in Fig. 2, the crotch member 30 includes a crotch inner sheet (inner sheet) 28 lying on the skin-facing surface, a crotch outer sheet (outer sheet) 29 lying on the non-skin-facing surface and a liquid-absorbent structure 80 interposed between the crotch inner sheet 28 and the crotch outer sheet 29. A dimension in the thickness direction W of the crotch member 30 is preferably in a range of about 0.5 to about 9.0mm and more preferably in a range of about 0.5 to about 5.0mm.

As material for the crotch inner sheet 28, well known liquid-permeable sheets such as air-through nonwoven fabrics, spun bonded nonwoven fabrics, hydrophilized perforated plastic films or laminates of such nonwoven fabrics and plastic films may be used.

A dimension of the crotch inner sheet 28 in the transverse direction X is larger than that of the liquid-absorbent structure 80 in the transverse direction X and, as illustrated in Fig. 4, opposite laterals 28a extending outward in the transverse direction X beyond the liquid-absorbent structure 80 are, for example, folded onto the non-skin-facing surface of the liquid-absorbent structure 80. In addition, a dimension of the crotch inner sheet 28 in the longitudinal direction Y is larger than a dimension of the liquid-absorbent structure 80 in the longitudinal direction Y and, in consequence, has extensions 28b extend in the longitudinal direction Y beyond the liquid-absorbent structure 80. In this way, the crotch inner sheet 28 covers entirely the skin-facing surface of the liquid-absorbent structure 80.

The crotch outer sheet 29 may be formed of known materials so long as they are poorly liquid-permeable or liquid-impermeable. For example, spun bonded/melt blown/spun bonded (SMS) fibrous nonwoven fabrics, air-through fibrous nonwoven fabrics, point bonded fibrous nonwoven fabrics, span bonded fibrous nonwoven fabrics or laminates of such nonwoven fabrics and plastic films may be used. The crotch outer sheet 2 9 according to this embodiment uses a laminate of fibrous nonwoven fabric 29a and plastic film 29b is used. The plastic film 29b to be used is preferably liquid-impermeable. And the plastic film 29b to be used is preferably clear and colorless.

According to this embodiment, both dimensions in the transverse direction X and the longitudinal direction Y of the crotch outer sheet 29 are larger than the corresponding dimensions of the crotch inner sheet 28 and the liquid-absorbent structure 80. Consequently, the crotch outer sheet 29 extends outward in the longitudinal direction Y beyond the liquid-absorbent structure 80 to opposite ends 31, 32 and extends outward in the transverse direction X beyond the liquid-absorbent structure 80 to opposite side edges 33. The crotch member 30 has a pair of side flaps 38 extending along the opposite edges 33 and a pair of end flaps 35, 36 extending along the opposite ends 31, 32 wherein these flaps 38, 35, 36 are defined, for example, by the crotch outer sheet 29.

The crotch outer sheet 29 has a pair of fold lines 39 extending in the longitudinal direction Y along the opposite side edges 33 and opposite laterals are folded inward onto the skin-facing surface of the crotch inner sheet 28 along the fold lines 39 to define the side flaps 38 and a rectangular midsection 37 extending between the side flaps 38 as viewed in the transverse direction X. As illustrated in Figs. 3 and 4, the plastic film 29b lies on the skin-facing surface and the fibrous nonwoven fabric 29a lies on the non-skin-facing surface in the midsection 37. In the side flaps 38, the fibrous nonwoven fabric 29a lies on the skin-facing surface and the plastic film 29b lies on the non-skin-facing surface.

Referring to Fig. 2, in the right side flap 38, the fibrous nonwoven fabric 29a in the vicinity of the rear end 32 of the opposite ends 31 is shown to be partially cutaway to expose leg elastic members (elastic members) 50, 60 and, in the left side flap 38, the fibrous nonwoven fabric 29a in the crotch region 4 is shown to be partially cutaway so that the leg elastic members 50, 60 may be partially exposed. Along the opposite side flaps 38 defined by the crotch outer sheet 29, a pair of the first leg elastic members 50 and a pair of the second leg elastic members 60 are disposed between the fibrous nonwoven fabric 29a and the plastic film 29b symmetrically about the longitudinal imaginary center line P, respectively.

As the first and second leg elastic members 50, 60 strand- or string-like elastic members may be used. The first and second leg elastic members 50, 60 are secured under tension to at least one of the fibrous nonwoven fabric 29a and the plastic film 29b with hot melt adhesive (not shown). In this regard, to secure the elastic members 50, 60 to the fibrous nonwoven fabric 29a and the plastic film 29b, instead of coating the elastic members 50, 60 with the adhesive, at least one of the fibrous nonwoven fabric 29a and the plastic film29bmaybe coated with the adhesive, or instead of coating the fibrous nonwoven fabric 29a and/or the plastic film 29b with the adhesive, the elastic members 50, 60 may be coated with the adhesive, or not only the elastic members 50, 60 but also the fibrous nonwoven fabric 29a and the plastic film 29b may be coated with the adhesive. In this diaper 1, along each of the side edges 33 of the crotch member 30, a plurality of the first leg elastic members 50 and a plurality of the second leg elastic members 60 are provided.

On the opposite side flaps 38 of the diaper 1, the opposite pairs of the first leg elastic members 50 curve to close the distance therebetween on the transverse imaginary center line Q, then to be gradually spaced from each other as they draw apart from the transverse imaginary center line Q until the respective pairs of the first and second leg elastic members 50, 60 extend in parallel to each other in the vicinity of the opposite ends 31, 32.

With the diaper put on the wearer's body, the first leg elastic members 50 contract around the wearer's thighs to prevent body waste such as urine from leaking around the thighs.

The second leg elastic members 60 rectilinearly extend in the longitudinal direction Y. These paired second leg elastic members 60 are in parallel to each other and also to the longitudinal imaginary center line P. These second leg elastic members 60 lie between the longitudinal imaginary center line P and the associated first leg elastic members 50.

With the diaper put on the wearer's body, the second leg elastic members 60 contract in the wearer's crotch region so as to space upward the side flaps 38 from the crotch inner sheet 28 and thereby to prevent body waste such as urine and feces from leaking sideways in the transverse direction X of the diaper 1.

Fig. 5 is a perspective view illustrating how to join the crotch member 30 to the waist member 10. The crotch member 30 and the rear panel 12 of the waist member 10 are bonded to each other with first adhesive 74 applied to at least one of the crotch member 30 and the rear panel 12. While the case in which only the crotch member 30 is coated with the first adhesive 74 to join the crotch member 30 is bonded to the rear panel 12 is described in this embodiment, it is possible to coat the rear panel 12 only with the first adhesive 74 or to coat both the rear panel 12 and the crotch member 30 with the first adhesive 74, respectively.

An adhesive coated region 70 provided by coating the crotch member 30 with the first adhesive 74 such as hot melt adhesive includes a first sub-region 71 extending in the transverse direction X in the midsection 37, a pair of second sub-regions 72 formed on the side flaps 38 so as to be spaced from opposite side edges 71a of the first sub-region 71 in the direction drawing apart from the longitudinal imaginary center line P and rectilinearly extending on the side flaps 38 and a pair of third sub-regions 73 extending from respective ends 72a in the longitudinal direction of the second sub-regions 72 toward the transverse imaginary center line Q. The first adhesive 74 with which the crotch member 30 is bonded to the rear panel 12 is provided to extend continuously in the longitudinal direction Y on the side flaps 38 and to extend continuously in the transverse direction X along the rear end 32 of the opposite ends 31, 32 lying in the rear waist region 3 so that a rear pocket 75 may be defined between the waist member 10 and the crotch member 30 when the waist member 10 and the crotch member 30 are bonded to each other. In this way, this rear pocket 75 can collect body waste such as feces when the wearer is in a lying posture and an intake capacity of the diaper may be correspondingly increased. In addition, the skin-facing surface of the crotch member 30 is bonded to the non-skin-facing surface of the waist member 10 in this diaper 1 and the body waste intake capacity may be further increased.

As illustrated in Fig. 2, an adhesive coated region 70a in the front waist region 2 to be coated with the first adhesive 74 with which the crotch member 30 is bonded to the front panel 11 is similar to the adhesive coated region 70 except that each of the third sub-regions 73 consists of a sub-region 73a spaced from the transverse imaginary center line Q and a sub-region 73b close to the transverse imaginary center line Q. The crotch member 30 is bonded to the waist member 10 so as to define a front pocket 76 between the waist member 10 and the crotch member 30.

As illustrated in Figs. 4 and 6, the liquid-absorbent structure 80 includes a first sheet 81 lying on the skin-facing surface, a second sheet 82 lying on the non-skin-facing surface and a liquid absorbent material 83 lying between the first and second sheets 81, 82. In this regard, in Fig. 4, size of absorbent polymer particles 84 is exaggeratingly illustrated to clarify the presence thereof.

The first sheet 81 is formed of a hydrophilized water-permeable fibrous nonwoven fabric made of thermoplastic synthetic resin. For example, the first sheet 71 is formed of a hydrophilized SMS fibrous nonwoven fabric made of polypropylene. More specifically, used is the hydrophilized water-permeable SMS fibrous nonwoven fabric having a mass per unit area in a range of about 10.0 to about 12.0g/m² composed of two spun bonded nonwoven fabric layers each having a mass per unit area in a range of about 4.0 to about 5.0g/m² and a melt blown nonwoven fabric layer having a mass per unit area in a range of about 0.5 to about 2.0g/m² interposed between these two spun bonded nonwoven fabric layers.

The second sheet 82 is formed of a hydrophobized or water repellent finished water-permeable or poorly water-permeable fibrous nonwoven fabric made of thermoplastic synthetic resin. For example, the second sheet 82 is formed of a hydrophobized or water repellent finished SMS fibrous nonwoven fabric made of polypropylene. More specifically, used is the hydrophilized poorly water-permeable SMS fibrous nonwoven fabric having a mass per unit area in a range of about 10.0 to about 13.0g/m² composed of two spun bonded nonwoven fabric layers each having a mass per unit area in a range of about 4.0 to about 6.0g/m² and a melt blown nonwoven fabric layer having a mass per unit area in a range of about 0.5 to about 2.0g/m² interposed between these two spun bonded nonwoven fabric layers. In this regard, it is possible to form the second sheet 82 with use of the same material as that of the first sheet 81 and in the same manner as the manner in which the first sheet 81 is formed.

Preferably, the liquid absorbent material 83 contains none of absorbent fibers and only the absorbent polymer particles 84. The absorbent polymer particles 84 maybe selected from various kinds of known polymers such as starch-based, acrylic acid-based and amino acid-based particulates and fibrous polymers which are water-insoluble and water swellable and have an absorption capacity of several dozen times of own mass. The mass per unit area of the absorbent polymer particles 84 used here is preferably in a range of about 60 to about 250g/m².

The liquid-absorbent structure 80 includes, as illustrated in Fig. 6, a plurality of absorbing regions 85 containing the absorbent polymer particles 84 between the first and second sheets 81, 82 and being alternately arranged in the longitudinal direction Y, and sealing regions 86 surrounding the absorbing regions 85.

In the respective absorbing regions 85, as illustrated in Fig. 4, the first sheet 81 and the second sheet 82 are spaced from each other and the second sheet 82 is coated, for example, with second adhesive 87 such as hot melt adhesive with which the absorbent polymer particles are secured to the second sheet 82. The second adhesive 87 may be applied, for example, to the entire area of the second sheet 72. In this diaper 1, eight absorbing regions 85 are arranged. In this regard, it is not essential to use the second adhesive 87 in the absorbing regions 85. Amass per unit area of the second adhesive 87 is preferably in a range of about 1 to about 10g/m².

In the respective sealing regions 86, as illustrated in Fig. 4 and Fig. 7, the first sheet 81 is coated with third adhesive 88 such as hot melt adhesive with which the first sheet 81 and the second sheet 82 are bonded to each other. The third adhesive 88 is applied, for example, to the entire area of the sealing regions 86. A mass per unit area of the third adhesive 88 is preferably in a range of about 3 to about 20g/m² and preferably larger than the mass per unit area of the second adhesive 87.

A dimension in the longitudinal direction Y of each of the absorbing regions 85 is preferably in a range of about 25 to about 100mm and a dimension in the longitudinal direction Y of each of the sealing regions 86 is preferably in a range of about 5 to about 30mm. In addition, an area ratio of the respective sealing regions 86 to the respective absorbing regions is preferably about 25% or less in order that body waste such as urine and feces may be reliably absorbed and retained by the liquid absorbent material 83 in any one or more absorbing regions 85 irrespectively of the region or regions onto which body waste is discharged.

The liquid-absorbent structure 80 is secured, as illustrated in Fig. 4, with fourth adhesive 91 such as hot melt adhesive provided between the crotch inner sheet 28 and the first sheet 81 and fifth adhesive 92 such as hot melt adhesive provided between the crotch outer sheet 29 and the second sheet 82. The fifth adhesive 92 is provided only in the vicinity of the transverse imaginary center line Q and the front and rear waist regions 2, 3 without providing the fifth adhesive in the remaining region in the longitudinal direction Y to prevent the dimension in the thickness direction W from increasing due to the fifth adhesive 92.

In this diaper 1, the absorbing regions 85 and the sealing regions 86 are arranged alternately in the longitudinal direction Y and, in the sealing regions 86, the first sheet 81 and the second sheet 82 are bonded to each other to prevent the absorbent polymer particles 84 from moving and thereby to prevent the absorbent polymer particles 84 from locally gathering together. Thereby it is possible to prevent the dimension W of the crotch member 30 in the thickness direction from increasing and to maintain the total luminous transmittance as measured from the crotch outer sheet 29 through the liquid-absorbent structure 80 to the crotch inner sheet 28 in a range of about 15% to about 65%. In consequence, feces discharged on the skin-facing surface of the crotch inner sheet 28 may be visually recognized through the crotch outer sheet 29. In this way, it is easily possible to perceive occurrence of defecation from the outside. The total luminous transmittance is preferably in a range of about 25% to about 35%.

In addition, the absorbent polymer particles 84 are secured to the first sheet 81 with the second adhesive 87 in the respective absorbing regions 85 so that the number of the absorbent polymer particles 84 in the liquid absorbent material 83 as counted in the thickness direction W may be maintained to be substantially uniform in the longitudinal direction as well as in the transverse direction. In this way, movements of the absorbent polymer particles 84 may be reliably prevented and thereby the total luminous transmittance may be improved. In consequence, it is further facilitated to perceive occurrence of defecation from the outside.

In the liquid-absorbent structure 80, a mass per unit area of the third adhesive is set to be larger than a mass per unit area of the second adhesive 87 so that the first sheet 81 and the second sheet 88 in the respective sealing regions 86 may be reliably bonded, on one hand, the absorbent polymer particles 84 are secured to the first sheet 81 and thereby the dimension of the second adhesive 87 in the thickness direction W may be prevented from increasing. In this way, the total luminous transmittance may be further improved.

For the waist outer sheet 21 and the crotch inner and outer sheets 28, 29, white and unpatterned sheets are used so that a difference between color of feces and white of the sheets 21, 28, 29 facilitates occurrence of defecation to be easily perceived from the outside.

The liquid-absorbent structure 80 constructed in this manner has the dimension W in the thickness direction is in a range of about 0.5mm to about 9.0mm and a bending resistance in the longitudinal direction Y is about 15 to about 140mm. The bending resistance was measured in accordance with Method A (45° cantilever method) prescribed in JIS L 1096, Section 8.19.1. Test pieces (having a dimension in the transverse direction X of 50mm and a dimension in the longitudinal dimension in the longitudinal direction of 50mm) including no fold line was cut out with use of scissors from the liquid-absorbent structures 80 in the vicinity of the transverse imaginary center line Q and the measurement was conducted three times. Three measurement results obtained are averaged to the bending resistance.

It should be appreciated that the absorbent article may be appropriately modified or varied without departure from the scope of this invention and not limited to the embodiment as having described hereinabove. For example, the absorbent article is not limited to the diaper 1 but also applicable to other absorbent articles such as toilet-training pants and the like. Further, the absorbent article is not limited to the diaper 1 of pants-type but also applicable to diapers of other types such as open-type. The constituent members of the diaper 1 are not limited to those described in the specification but the other various types of materials widely used in the relevant technical field may be used without limitation unless otherwise stated.

In some embodiments described hereinabove, the crotch outer sheet 29 alone has the side flaps 38 and the front and rear end flaps 35, 36. However alternative arrangements are alsopossible such that the crotch inner sheet 28 alone has the side flaps 38 and the front and rear end flaps 35, 36 or both the crotch inner sheet 28 and the crotch outer sheet 29 have the side flaps 38 and the front and rear end flaps 35, 36.

In some embodiments described hereinabove, the sealing regions 86 of the liquid-absorbent structure 80 contain none of the absorbent polymer particles 84. However, the sealing regions 86 may contain a small amount of the absorbent polymer particles 84 mixing in these regions 86 in the course of the manufacturing process. In other words, In this invention, the term "sealing regions 86" refers to the regions in which none of the absorbent polymer particles is present and the regions in which a certain amount of the absorbent polymer particles are present but substantially none of the absorbent polymer particles is present.

In some embodiments described hereinabove, the absorbing regions 85 of the liquid-absorbent structure 80 contain the absorbent polymer particles 84 alone. However, the absorbing regions 85 may contain, in addition to the absorbent polymer particles 84, absorbent fibers. As the absorbent fibers, natural pulp fibers such as wood fluff pulp, regenerated fibers such as rayon fibers or amixture thereof maybe used. When the absorbent fibers are added to the absorbent polymer particles 84, a ratio by mass of the absorbent polymer particles 84 to the absorbent fibers is preferably in a range of 7:3 to 10:0.

In some embodiments described hereinabove, the sealing regions 86 are coated over the entire area thereof with the third adhesive 88. However, this invention is not limited to such embodiment and it is also possible to coat the portions only of the sealing regions 86 corresponding to the peripheral edges of the absorbing regions with the third adhesive 88 so that the sealing regions 86 may include the adhesive-free portions in the longitudinal direction Y to improve the total luminous transmittance in these portions.

### {Examples}

In the crotch member 30 including factors as described below, a total luminous transmittance from the crotch outer sheet 29 through the liquid-absorbent structure 80 to the crotch inner sheet 28 was measured.

A dimension in the thickness direction W of the crotch member 30 was 0. 9mm. As the crotch inner sheet 28, a hydrophilized spun bonded nonwoven fabric having a mass per unit area of 18g/m² was used. As the crotch outer sheet 29, a laminate of a hydrophobized SMS fibrous nonwoven fabric 29a having a mass per unit area of 11g/m² and an unpatterned white moisture-permeable plastic film 29b having a mass per unit area of 18g/m² was used. In the liquid-absorbent structure 80, a hydrophilized SMS fibrous nonwoven fabric having a mass per unit area of 10g/m² was used as the first sheet 81 and a hydrophobized SMS fibrous nonwoven fabric having a mass per unit area of 11g/m² was used as the second sheet 82. The second adhesive 87 used to secure the absorbent polymer particles 84 to the second sheet 82 was applied to the entire area of the second sheet 82 at a rate of 3g/m² and the third adhesive 88 used to bond the first sheet 81 and the second sheet 82 to each other was applied to the first sheet 81 of the sealing regions 86 at a rate of 10g/m². A dimension in the thickness direction W of the respective absorbing regions 85 was 0.9mm and a dimension in the thickness direction W of the sealing regions 86 was 0.7mm.

Test pieces having a dimension in the transverse direction X of 50mm and a dimension in the longitudinal direction Y of 50mm were cut out from the crotch members 30 with use of scissors and a total luminous transmittance from the crotch outer sheet 29 through the liquid-absorbent structure 80 to the crotch inner sheet 28 were measured three times with use of A300/ZE-2000 manufactured by Nippon Denshoku Industries CO., LTD. in Japan and an average value was calculated. The result is indicated in TABLE 1.

**{TABLE 1}**

| | Total luminous transmission (%) |
|---|---|
| Example 1-A | 26.9 |
| Example 1-B | 28.8 |
| Comparative Example 1 | 10.2 |
| Comparative Example 2 | 13.1 |

In TABLE 1, Example 1-A indicates an average value of the total luminous transmittance of the absorbing regions 85 in the crotch member 30 of this diaper 1 and Example 1-B indicates an average value of the total luminous transmittance of the sealing regions 86 in the crotch member 30.

Comparative Example 1 indicates an average value of the total luminous transmittance of the crotch member including the crotch inner sheet, the crotch outer sheet and the liquid-absorbent structure and having a dimension in the thickness direction of 5.8mm. The liquid-absorbent structure of this crotch member is formed of absorbent polymer particles and absorbent fibers and provided with a continuous core member extending in the longitudinal direction.

Comparative Example 2 indicates an average value of the total luminous transmittance of the crotch member including the crotch inner sheet, the crotch outer sheet and the liquid-absorbent structure and having a dimension in the thickness direction of 3.2mm. The liquid-absorbent structure of this crotch member is formed of absorbent polymer particles and absorbent fibers and provided with a continuous core member extending in the longitudinal direction.

### {Reference Signs List}

- 1: diaper (absorbent article)
- 10: waist member
- 28: crotch inner sheet (inner sheet)
- 29: crotch outer sheet (outer sheet)
- 30: crotch member
- 31: front end
- 32: rear end
- 33: opposite side edges
- 35: front end flap
- 36: rear end flap
- 38: side flaps
- 80: liquid-absorbent structure
- 81: first sheet
- 82: second sheet
- 84: liquid-absorbent polymer particles
- 85: absorbing region
- 86: sealing region
- X: transverse direction
- Y: longitudinal direction
- W: thickness direction

## Claims

1. A disposable absorbent article comprising an inner sheet lying on a skin-facing surface, an outer sheet lying on a non-skin-facing surface and a liquid-absorbent structure interposed between these inner and outer sheets, wherein:
a total luminous transmittance from the outer sheet through the liquid-absorbent structure to the inner sheet is in a range of 15% to 65%.

2. The absorbent article defined by claim 1, wherein the liquid-absorbent structure has a longitudinal direction, a transverse direction and a thickness direction and includes:
absorbing regions containing absorbent polymer particles between a first sheet and a second sheet and being arranged alternately in the longitudinal direction; and
sealing regions surrounding each of the absorbing regions and being defined by sealing the first and second sheet.

3. The absorbent article defined by claim 2, wherein the liquid-absorbent structure has a dimension in,the thickness direction in a range of 0.5mm to 9.0mm and has a bending resistance in the longitudinal direction measured by a cantilever method is in a range of 15 to 140mm.

4. The absorbent article defined by claim 3, wherein the absorbent article is provided in the form of pants-type including an annular waist member, the inner sheet, the outer sheet, the liquid-absorbent structure and a crotch member bonded to the non-skin-facing surface of the waist member.

5. The absorbent article defined by claim 4, wherein: the crotch member includes a pair of side flaps and a pair of front and rear end flaps respectively extending along opposite side edges and opposite ends of at least one of the inner sheet and the outer sheet and the side flaps are folded onto the skin-facing surface; and
the side flaps are continuously coated in the longitudinal direction with adhesive with which the crotch member is bonded to the waist member and thereupon a rear pocket may be formed between the waist member and the crotch member.

6. The absorbent article defined by claim 5, wherein an unpatterned white fibrous nonwoven fabric is used as a material for the inner sheet and the outer sheet and the unpatterned white fibrous nonwoven fabric is used also as a material for a waist outer sheet lying on the non-skin-facing surface.
